# EUROPEAN PATENT APPLICATION

(11) **EP 4 813 447 A1**
(43) Date of publication of application: **30.09.2026**
(21) Application number: 26166914.7
(22) Date of filing: 23.03.2026
(51) Int. Cl.: A61N 5/06, B05B 1/18

(54) **A MULTIFUNCTIONAL SHOWER HEAD ASSEMBLY**

(30) Priority: 25.03.2025 CN 202520531186 U
(71) Applicant: Shenzhen Rainbow Light Medical Equipment Co., Ltd., Shenzhen, Guangdong 518103 (CN); Higherdose LLC, New York, NY 10004 (US)
(72) Inventor: BERLINGERI, Lauren, New York, 10004 (US); DIJKSTRA, Alain, 1186 GK Amstelveen (NL); JIAMING, Cao, Shenzhen, 518103 (CN); XIANG, Huang, Shenzhen, 518103 (CN); ZUN, Qui, Shenzhen, 518103 (CN); GUANG, Tian, Shenzhen, 518103 (CN)
(74) Representative: Valet Patent Services Limited

(57) **Abstract**

The present invention discloses a multifunctional shower head incorporating a detachable phototherapy unit configured to deliver therapeutic irradiation during normal shower use. The device includes a shower body with an inlet section, an outlet section, and an annular water-dispensing ring provided with multiple outlet holes. A phototherapy panel is mounted on the shower head and includes an LED lamp board, lenses, a sealed cover plate, and a waterproof housing. The phototherapy panel is detachably coupled to the outlet section through a locking interface, allowing independent installation, removal, and maintenance. The external housing may be square, round, or other shapes without affecting internal functionality. The system further includes a wireless remote-control unit for adjusting light modes, intensity, and therapy duration. This configuration enables simultaneous delivery of therapeutic light and water, improving convenience, safety, and overall user experience.

## Description

### TECHNICAL FIELD

The present invention relates generally to shower systems and personal-care equipment, and more particularly to a multifunctional shower head incorporating an integrated or detachable phototherapy unit. The invention further relates to shower devices configured to deliver therapeutic light, water-spray functionality, and control features through a modular structure. Additionally, the invention pertains to shower assemblies capable of supporting variable external shapes and wireless remote-control operation.

### BACKGROUND ART

Traditional shower heads are generally designed to deliver water in a fixed spray pattern without offering any additional skincare or therapeutic functionality. Conventional designs focus primarily on water pressure, spray distribution, and ergonomic handling. While incremental improvements such as adjustable spray modes or removable filters have been introduced, these features do not provide any targeted skin-treatment benefits during regular bathing. As a result, consumers seeking cosmetic or therapeutic light treatments typically rely on separate handheld phototherapy devices, which must be used before or after showering. This separation creates inconvenience, increases treatment time, and reduces user compliance.

Furthermore, most available phototherapy devices are not waterproof and therefore cannot be safely used in wet environments, restricting their utility during bathing when the skin is already hydrated and receptive to treatment. Existing shower heads also lack any integrated mechanism for controlled, safe illumination due to concerns regarding electrical exposure, sealing, and durability.

The present invention addresses these limitations by integrating a detachable phototherapy unit directly into the shower head. The system provides safe, waterproof light delivery through a sealed annular housing that encloses the light-emitting module, power components, and control circuitry. The detachable configuration allows easy maintenance, replacement, and optional usage, enabling users to enjoy either a conventional shower or a combined shower-plus-phototherapy treatment.

By merging water delivery with therapeutic illumination, the invention significantly enhances user convenience, reduces treatment time, and ensures consistent exposure to beneficial wavelengths such as red, blue, or infrared light during showering. This integrated design overcomes the shortcomings of traditional shower heads and separate phototherapy devices, offering a compact, reliable, and multifunctional solution for both hygiene and skincare needs.

### OBJECTS OF THE INVENTION

Some of the objects of the invention are as follows:

An object of the present invention is to provide a multifunctional shower head that integrates both water-spraying functionality and therapeutic phototherapy within a single device.

Another object of the invention is to provide a shower head equipped with a detachable phototherapy unit, allowing users to easily install, remove, clean, or independently operate the phototherapy unit as desired.

Another object of the invention is to provide a waterproof phototherapy unit and sealed housing capable of preventing water ingress during high-pressure shower operation, thereby enhancing electrical safety and long-term reliability.

Another object of the invention is to provide a phototherapy unit capable of emitting therapeutic light within a broad 630-850 nm range and an optimized 650-850 nm range for enhanced skin treatment.

An object of the invention is to provide a wireless remote-control unit that enables users to adjust light modes, intensity levels, and therapy duration without touching the main device body, ensuring safe and convenient operation in wet environments.

Another object of the invention is to provide a shower head capable of adopting various external shapes, such as square, triangular, round, rectangular, or other geometries, without altering the functional performance of the phototherapy system or water-dispensing structure.

An object of the invention is to enhance the overall user experience by combining therapeutic light irradiation, adjustable water flow, and wireless control features in a unified, compact, and user-friendly shower assembly.

Yet another object of the invention is to provide a structure that is simple to manufacture, easy to maintain, and adaptable to different bathroom environments while maintaining robust sealing, stability, and therapeutic effectiveness.

### SUMMARY OF THE INVENTION

According to a first aspect of the present invention, a phototherapy unit configured for retrofit installation on a shower head is provided. The phototherapy unit comprises: a housing defining a central opening sized to receive an outlet portion of the shower head; a plurality of light-emitting elements disposed within the housing and oriented to emit therapeutic light toward a user during shower operation; and a locking mechanism disposed on the housing and configured to detachably secure the phototherapy unit onto an exterior surface of the shower head; wherein the phototherapy unit mounts to the shower head without structural modification of the shower head structure.

In one embodiment of the invention, the housing is selected from a circular, oval, polygonal, square, triangular or rectangular shape to accommodate shower heads having different geometries.

In one embodiment of the invention, the housing includes a region supporting a lamp board, lenses, and a transparent cover plate.

In one embodiment of the invention, the locking mechanism comprises at least one groove, slot, ridge, or tab configured to engage a complementary locking feature on the shower head.

In one embodiment of the invention, the light-emitting elements emit wavelengths in the range of 630 to 850 nm.

In one embodiment of the invention, the locking mechanism comprises engagement features configured for insertion and rotation to secure the phototherapy unit to the shower head.

In one embodiment of the invention, the phototherapy unit is detachable for charging or maintenance independently of the shower head.

According to a second aspect of the present invention, a shower head assembly is provided. The shower head assembly comprises: a shower head having an inlet portion, an outlet portion, and a plurality of water outlet holes located at a front face of the outlet portion; and a phototherapy unit detachably coupled to the outlet portion, the phototherapy unit including a housing surrounding the shower head; wherein the phototherapy unit comprises a plurality of light-emitting elements; wherein the shower head and the phototherapy unit are configured to deliver the phototherapy during a shower.

In one embodiment of the invention, the phototherapy unit includes a mounting shell defining a groove for seating the light-emitting elements.

In one embodiment of the invention, the outlet portion includes a connection position formed with a first guide groove, a second guide groove, and a locking groove.

In one embodiment of the invention, the phototherapy unit includes a locking protrusion configured to enter the first guide groove, travel into the second guide groove, and seat in the locking groove during rotational engagement.

In one embodiment of the invention, multiple locking protrusions and multiple connection positions are circumferentially arranged to increase mounting stability.

In one embodiment of the invention, the light-emitting elements are mounted on a metal-core circuit board for improved thermal management.

In one embodiment of the invention, the phototherapy unit includes one or more lenses aligned one-to-one with the light-emitting elements to focus emitted light.

According to a third aspect of the present invention, a phototherapy unit for a shower head is provided. The phototherapy unit comprises: a housing configured to be mounted on or integrated with an outlet portion of the shower head; a plurality of light-emitting elements disposed on the housing; a power supply electrically connected to the light-emitting elements; and a waterproofing structure enclosing the power supply and an electrical circuitry within the housing; wherein the waterproofing structure prevents water ingress into the electrical circuitry during shower operation; wherein the outlet portion includes a connection position formed with a first guide groove, a second guide groove, and a locking groove; wherein the phototherapy unit includes a locking protrusion configured to enter the first guide groove, travel into the second guide groove, and seat in the locking groove during rotational engagement.

In one embodiment, the housing enables axial insertion of the outlet portion followed by rotational locking.

In one embodiment, the transparent cover plate is formed of glass, polycarbonate, or silicone.

In one embodiment, the waterproofing structure comprises at least one sealing ring, gasket, adhesive bonding layer, ultrasonic-welded joint, or conformal coating applied to the lamp board.

In one embodiment, the power supply comprises a rechargeable battery enclosed within the waterproofing structure.

In one embodiment, the phototherapy unit further comprising a wireless charging coil disposed within the housing.

In the context of the specification, the terms "first", "second," and "third" are only used for descriptive purposes and do not imply the relative importance or implicitly indicate the quantity of technical features indicated.

In the context of the specification, the term "plurality" means two or more than two, unless otherwise indicated.

In the context of the specification, the term "several" means more than one, unless otherwise specified.

In the context of the specification, the term "phototherapy element" or "light-emitting element" encompasses any light-emitting device capable of emitting light of therapeutic wavelength(s), including but not limited to light-emitting diodes (LEDs), organic LEDs (OLEDs), laser diodes, or equivalent optical sources. The light may include ultraviolet, visible, near-infrared, or far-infrared spectra. The terms "light-emitting element," "light source," "light-emitting module," and "LED bead" may be used interchangeably throughout this specification.

In the context of the specification, the term "detachable" or "detachably mounted" refers to a connection or attachment that can be separated and reconnected without causing permanent damage to the components, and without requiring specialized tools or destructive disassembly. A detachable connection may include mechanical fasteners, snap-fit elements, threaded connections, bayonet mounts, magnetic attachments, or other reversible coupling mechanisms.

In the context of the specification, the term "waterproof" refers to the ability of a component or assembly to prevent water ingress under specified conditions of use, such as exposure to water spray, splashing, or temporary immersion. Waterproof performance may be characterized by an Ingress Protection (IP) rating, such as IPX4 (splash-resistant), IPX7 (immersion up to 1 meter for 30 minutes), or IPX8 (continuous immersion beyond 1 meter). The term encompasses sealing methods such as gaskets, O-rings, adhesive bonding, ultrasonic welding, over molding, or conformal coatings applied to electronic components.

In the context of the specification, the term "therapeutic light" or "phototherapy" refers to the application of electromagnetic radiation in the visible or near-infrared spectrum for the purpose of producing beneficial biological effects in human tissue. Therapeutic light may include wavelengths ranging from approximately 400 nm to 1400 nm, and may be used to stimulate cellular metabolism, enhance blood circulation, reduce inflammation, promote tissue repair, alleviate pain, or treat skin conditions. Phototherapy may be delivered through continuous or pulsed illumination at specified intensities and durations.

In the context of the specification, the term "annular" or "ring-shaped" refers to a structure having a circular or substantially circular outer boundary and a circular or substantially circular inner boundary, thereby forming a ring or toroidal configuration. An annular structure may have a uniform or variable cross-sectional profile, and may be formed as a complete ring or as a segmented ring with one or more gaps or openings.

In the context of the specification, the term "housing" or "housing structure" is intended to cover any casing, enclosure, or structural body that contains or supports components of the device. The housing may include a handle portion, head portion, or other segments, and may be made from polymeric, metallic, composite, or other suitable materials. The housing may be of any shape, including but not limited to circular, annular, square, triangular, rectangular, oval, polygonal, or irregular shapes, to accommodate different shower head geometries.

In the context of the specification, the terms "head" or "phototherapy head" refer to a portion of the device coupled to the housing and configured to emit light toward the skin. The head may include one or more light-transmitting surfaces, optical lenses, or diffusers, and may also support electrodes or other stimulation elements.

In the context of the specification, the term "circuit board" encompasses any printed circuit board (PCB), flexible circuit, or equivalent substrate that supports and electrically connects components of the device, including power supplies, control chips, drivers, or stimulation elements.

In the context of the specification, the term "LED module" refers to one or more light-emitting diode (LED) elements that are electrically connected and configured to emit light of specific wavelengths suitable for therapeutic purposes. The LED module may include drive circuitry, heat dissipation structures, and optical elements such as lenses or diffusers to control light distribution.

In the context of the specification, the term "light source" or "phototherapy source" etc. refers to a source emitting coherent laser light, or light-emitting diodes ("LEDs"). The term "light therapy" refers to light generated from any of the sources, such as lasers, LED sources, or Super luminous diodes ("SLD").

In the context of the specification, "Light Emitting Diodes (LEDs)" refer to semiconductor diodes capable of emitting electromagnetic radiation when supplied with an electric current. The LEDs are characterized by superior power efficiencies, smaller sizes, rapid switching speeds, physical robustness, and longer lifespans compared to incandescent or fluorescent lamps. The one or more LEDs may include through-hole type LEDs (generally emitting electromagnetic radiation in red, green, yellow, blue, and white colors), Surface Mount Technology (SMT) LEDs, Bi-color LEDs, Pulse Width Modulated RGB (Red-Green-Blue) LEDs, and high-power LEDs, among others.

Materials used in one or more LEDs may vary from one embodiment to another, depending upon the frequency of radiation required. Different frequencies can be obtained from LEDs made from pure or doped semiconductor materials. Commonly used semiconductor materials include nitrides of Silicon, Gallium, Aluminum, Boron, Zinc Selenide, etc., in pure form or doped with elements such as Aluminum and Indium. For example, red and amber colors are produced from Aluminum Indium Gallium Phosphide (AlGaInP) based compositions, while blue, green, and cyan use Indium Gallium Nitride based compositions. White light may be produced by mixing red, green, and blue lights in equal proportions, while varying proportions may be used to generate a wider color gamut. White and other colored lightings may also be produced using phosphor coatings such as Yttrium Aluminum Garnet (YAG) in combination with a blue LED to generate white light, and Magnesium-doped potassium fluorosilicate in combination with a blue LED to generate red light.

In addition to conventional mineral-based LEDs, one or more LEDs may also be provided on an Organic LED (OLED) based flexible panel or an inorganic LED-based flexible panel. Such OLED panels may be generated by depositing organic semiconducting materials over Thin Film Transistor (TFT) based substrates. Further, a discussion on the generation of OLED panels can be found in Bardsley, J. N (2004), "International OLED Technology Roadmap", IEEE Journal of Selected Topics in Quantum Electronics, Vol. 10, No. 1, that is included herein in its entirety, by reference. An exemplary description of flexible inorganic light-emitting diode strips can be found in granted U.S. Pat. No. 7,476,557 B2, titled "Roll-to-roll fabricated light sheet and encapsulated semiconductor circuit devices", which is included herein in its entirety by reference.

Unless otherwise stated, the term "light" as used in this specification encompasses electromagnetic radiation in the visible (380-780 nm) and infrared (780 nm-1000 nm) ranges, particularly red light (620-750 nm) and near-infrared (750-1400 nm) wavelengths commonly used in photobiomodulation therapy. Particular wavelengths which may be selected as the dominant emissive wavelength may include the follow, without any preference to be indicated by order: 400 nm, 405 nm, 420 nm, 430 nm, 450 nm, 465 nm, 515 nm, 530 nm, 532 nm, 590 nm, 630 nm, 633 nm, 640 nm, 650 nm, 655 nm, 660 nm, 670 nm, 680 nm, 780 nm, 785 nm, 810 nm, 830 nm, 840 nm, 850 nm, 860 nm, 870 nm, 904 nm, 915 nm, 980 nm, 1015 nm, 1060 nm, 1065 nm, 1070 nm, 1200, and 1400 nm. As used herein, the term "light therapy" refers to the use of one or more light sources of any type that emit light with a wavelength between about 400 and 1400 nm. The device may also emit blue or ultraviolet light for surface-level treatments such as acne reduction or microbial control.

The red light (approximately 630-660 nm) penetrates deeply into the scalp to stimulate blood circulation and enhance hair follicle activity, thus promoting hair growth and repair. Blue light (around 415-470 nm) exhibits antibacterial properties and is effective in treating scalp acne and reducing inflammation. Green light (approximately 520-540 nm) can help reduce pigmentation and soothe sensitive or irritated scalp tissue. Yellow light (around 580-600 nm) improves oxygen exchange in the cells and aids in detoxifying the scalp, while near-infrared light (800-850 nm) reaches deeper layers to accelerate healing and reduce pain.

### BRIEF DESCRIPTION OF THE ACCOMPANYING DRAWINGS

The accompanying drawings illustrate the best mode for carrying out the invention as presently contemplated and set forth hereinafter. The present invention may be more clearly understood from a consideration of the following detailed description of the preferred embodiments taken in conjunction with the accompanying drawings, wherein like reference letters and numerals indicate the corresponding parts in various figures in the accompanying drawings, and in which:
**FIG. 1** is a three-dimensional structural view of a multifunctional shower head, in accordance with an embodiment of the present invention.
**FIG. 2** is an exploded view illustrating the components of the multifunctional shower head shown in FIG. 1, in accordance with an embodiment of the present invention
**FIG. 3** is an exploded view of the shower head body depicted in FIG. 2, in accordance with an embodiment of the present invention.
**FIG. 4** is an exploded view of the phototherapy mechanism included in the multifunctional shower head of FIG. 2, in accordance with an embodiment of the present invention.
**FIG. 5** is a three-dimensional structural view of the mounting shell shown in the phototherapy mechanism of FIG. 4, in accordance with an embodiment of the present invention.
**FIG. 6** is a perspective view of another configuration of the shower head body, in accordance with an embodiment of the present invention.
**FIG. 7** is a perspective view of yet another configuration of the shower head body, in accordance with an embodiment of the present invention.
**FIG. 8** is a perspective view of a remote-control unit, in accordance with an embodiment of the present invention.

### DETAILED DESCRIPTION

Embodiments of the present invention disclosure will be described more fully hereinafter with reference to the accompanying drawings in which like numerals represent like elements throughout the figures, and in which example embodiments are shown.

The detailed description and the accompanying drawings illustrate the specific exemplary embodiments by which the disclosure may be practiced. These embodiments are described in detail to enable those skilled in the art to practice the invention illustrated in the disclosure. It is to be understood that other embodiments may be utilized, and other changes may be made, without departing from the spirit or scope of the present disclosure. The following detailed description is therefore not to be taken in a limiting sense, and the scope of the present invention disclosure is defined by the appended claims. Embodiments of the claims may, however, be embodied in many different forms and should not be construed as limited to the embodiments set forth herein.

The terms "a" and "an" herein do not denote a limitation of quantity, but rather denote the presence of at least one of the referenced items. The terms "having", "comprising", "including", and variations thereof signify the presence of a component.

Embodiments of the present invention disclose a multifunctional shower head integrating a detachable phototherapy mechanism and optional therapeutic enhancements.

In an embodiment, the multifunctional shower head comprises a shower head body and a detachable phototherapy unit. The shower head body includes a water inlet section and a water outlet section. The water inlet section is formed with a water inlet hole configured to receive water from an external pipe or hose. The water outlet section is connected to the inlet section and includes one or more water outlet holes through which water is discharged during shower operation. A filter can be disposed within the water inlet section to remove impurities, sediments, or contaminants from the incoming water. The filter can be removable or replaceable, and can include mesh filters, carbon-based filters, or ion-exchange elements.

The water outlet section includes a connection structure configured to detachably engage the phototherapy unit. In one embodiment, the connection structure comprises a first groove, a second groove, and a locking groove. The first groove may extend longitudinally along the axial direction of the outlet section, while the second groove extends circumferentially along a portion of the outlet section. The locking groove is positioned to receive a locking protrusion of the phototherapy unit when the unit is rotated or slid into place, thereby achieving a stable mechanical engagement.

The phototherapy unit includes a central mounting hole sized and shaped to receive the water outlet section. A locking protrusion or locking slot is disposed on the inner wall of the mounting hole. When the phototherapy unit is mounted, the outlet section is inserted through the mounting hole, aligned with the first groove, rotated to engage the second groove, and finally seated into the locking groove, providing a secure but detachable connection.

The shower head body may further include a water distribution cover, a center cover, and a snap or buckle cover mounted near the outlet end. These elements function to define internal water channels, regulate the pressure and pattern of the water stream, and stabilize the flow as it exits the water outlet holes. The covers may be attached by threaded connection, snap-fit connection, or screw fasteners.

The phototherapy unit comprises a housing that includes an annular mounting shell and a light-transmitting cover plate. The mounting shell defines an internal annular groove configured to accommodate electronic components, including a lamp board, lenses, battery, control circuitry, and optional support structures. The light-transmitting cover plate is disposed over the annular groove and may be sealed to the mounting shell to provide waterproof protection.

A lamp board is positioned within the annular groove. The lamp board includes multiple LED beads (light-emitting elements) arranged in a circular or annular configuration surrounding the central mounting hole. Each LED bead may correspond one-to-one with a respective lens disposed between the LED bead and the cover plate. The lenses focus, diffuse, or otherwise modify the light output to improve therapeutic penetration and user comfort.

The LED beads (light-emitting elements) may emit therapeutic wavelengths such as red light, blue light, near-infrared light, or combinations thereof. In some embodiments, different LEDs may provide different wavelengths simultaneously or alternately.

In some embodiments, the water outlet holes of the shower head body are arranged in alternating positions with the LED beads (light-emitting elements) of the phototherapy unit. In such configurations, the phototherapy unit emits light between adjacent water streams or through transparent sections between the outlet holes, allowing therapeutic light to merge with the water flow. This arrangement enables light to reach the user's skin while water is being delivered, improving the efficiency and convenience of combined showering and phototherapy treatments.

The phototherapy unit includes a rechargeable battery disposed within the housing. The battery is electrically connected to the lamp board, a control circuit, and a user interface button. The control button is mounted on the housing and covered by a flexible, waterproof keycap to ensure safe operation.

A charging interface may be provided through a sealed through-hole. A charging port is mounted within the through-hole and protected by a sealing cover. In alternative embodiments, the phototherapy unit incorporates externally accessible charging electrodes or includes a wireless charging coil enabling inductive charging through a charging dock.

An internal annular bracket or support frame may be installed to stabilize the lamp board, lenses, battery, and associated circuitry.

During use, the user may attach the phototherapy unit by inserting the shower head outlet section into the mounting hole and rotating the unit until the locking protrusion engages the locking groove. Water entering the inlet section passes through the filter, flows through the distribution covers, and exits the outlet holes. Simultaneously, the user may activate the phototherapy unit by pressing the waterproof button, causing the LEDs to emit therapeutic light.

When phototherapy is not desired, the phototherapy unit may be removed, and the shower head used conventionally.

Several embodiments of the present invention will now be described in detail with references to **FIGS.**

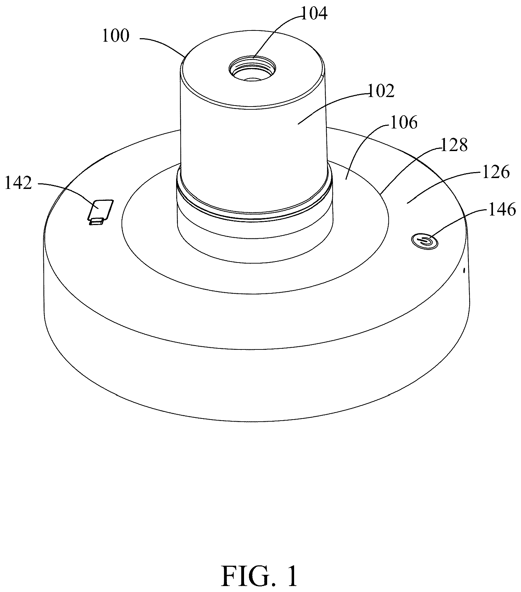

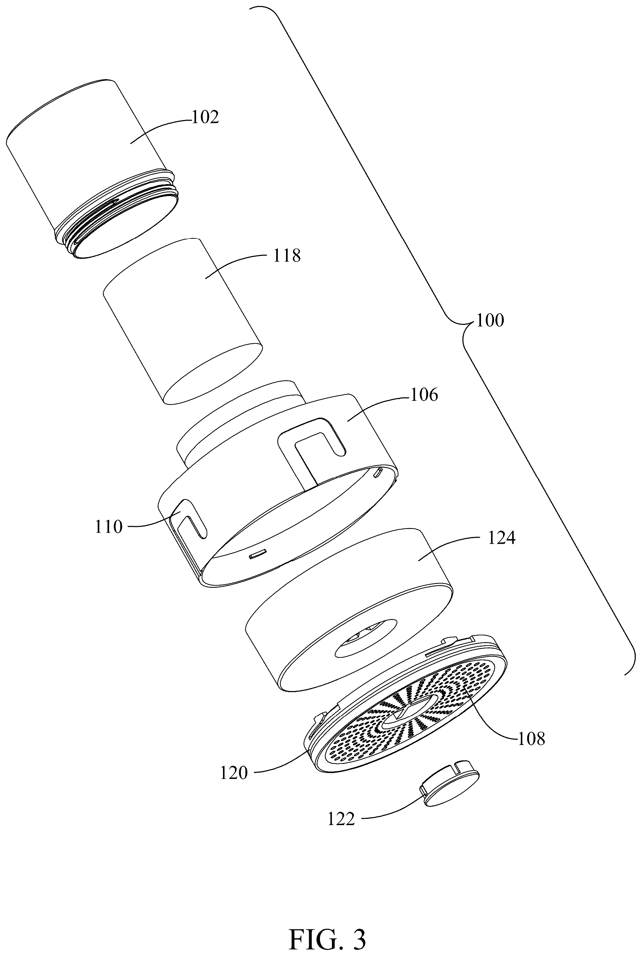

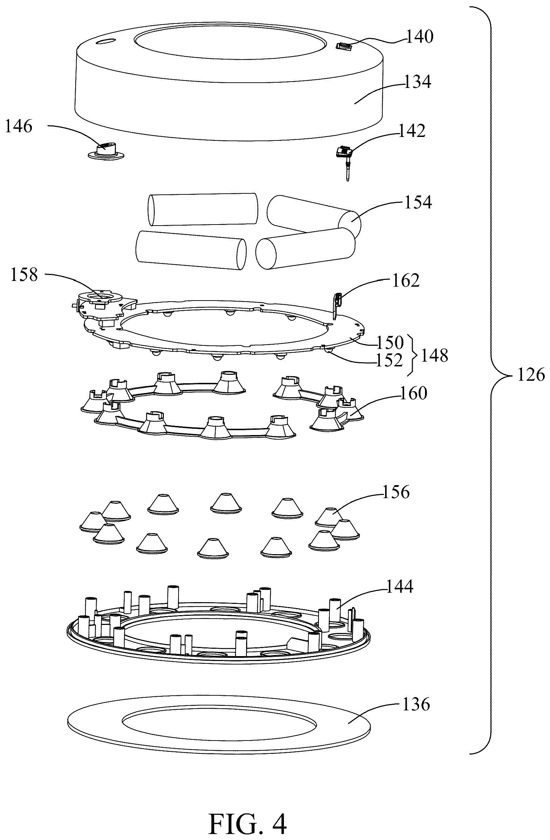

Referring now to **FIGS. 1 to 4** collectively, a multifunctional shower head according to the embodiments of the present application is described in detail. As shown, the multifunctional shower head comprises a shower body 100 and a phototherapy unit 126. The shower body 100 includes an inlet section 102 and an outlet section 106. One end of the inlet section 102 is provided with an inlet hole 104, and one end of the outlet section 106 is connected to the opposite end of the inlet section 102. The distal end of the outlet section 106 is provided with an outlet hole 108, and the outer peripheral surface of the outlet section 106 is formed with a connecting position 110.

The phototherapy unit 126 is configured to perform phototherapy on a user. A mounting hole 128 is formed at the center of the phototherapy unit 126 for receiving insertion of the outlet section 106. An inner wall of the mounting hole 128 is provided with a locking position 132, which is structured to engage the connecting position 110 to achieve a detachable locking connection. It should be noted that the inlet section 102 and the outlet section 106 may be integrally formed as a single structure or may be fabricated as two components that are detachably coupled. In either case, the inlet section 102 is in fluid communication with the outlet section 106. During use, water enters the inlet section 102 through the inlet hole 104, flows into the outlet section 106, and is discharged through the outlet hole 108. The inlet section 102 and the outlet section 106 may be arranged at a predetermined angle, such as a right angle, an acute angle, or may alternatively be coaxially aligned.

The phototherapy unit 126 comprises a light source (also referred to as light-emitting elements or light-emitting module) capable of generating therapeutic light. In an embodiment, the phototherapy unit 126 may emit red light to alleviate pain, blue light to reduce inflammation, infrared light to promote circulation, or selected wavelengths of therapeutic light according to user preference. This enables customized phototherapy functionality integrated within the showering process.

The phototherapy unit 126 and the outlet section 106 are detachably connectable. For assembly, the outlet section 106 is inserted into the mounting hole 128, and the locking position 132 cooperates with the connecting position 110 to form a secure yet detachable engagement. This arrangement simplifies assembly and disassembly of the phototherapy unit 126, facilitates manufacturing and sealing of internal circuits within the phototherapy unit 126, and enhances overall electrical safety.

By integrating the phototherapy unit 126, the user can simultaneously perform showering and phototherapy, thereby improving the functional performance of the shower body 100 without requiring separate phototherapy sessions. This improves convenience and enhances the overall showering experience.

Since the phototherapy unit 126 is locked to the outlet section 106 via the locking position 132 and the connecting position 110, the multifunctional shower head can be manufactured efficiently, and the phototherapy unit 126 can be conveniently removed for maintenance or independent use when desired. In certain optional embodiments, the outlet hole 108 may be provided in multiple quantities, disposed in a multi-ring array configuration. Such an arrangement allows the discharged water to be evenly distributed, thereby improving water dispersion performance.

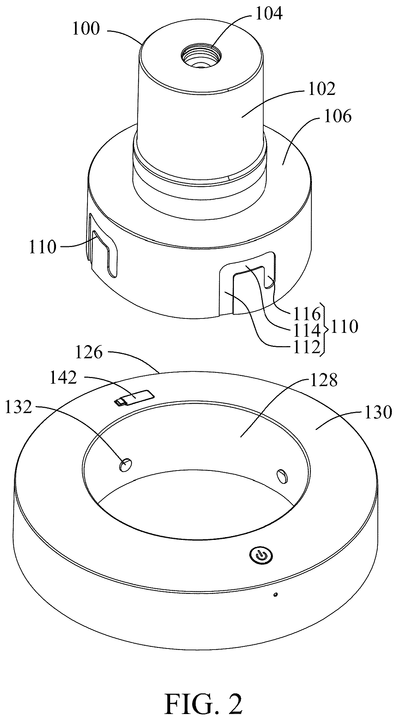

Referring to **FIGS. 2** to **4****,** in an embodiment, the phototherapy unit 126 comprises a housing 130, a light source 148 (also referred to as light-emitting elements or light-emitting module), and a battery 154. The light source 148 is disposed within the housing 130, and the battery 154 is configured to provide electrical power for driving the light source 148. The battery 154 is likewise disposed within the housing 130 and is electrically connected to the light source 148. By integrating the battery 154 into the housing 130, the light source 148 can be powered without the need for external wiring, thereby enhancing waterproof performance and improving the operational safety of the phototherapy unit 126.

In one embodiment, the light source 148 (light-emitting elements) includes a lamp board 150 and a plurality of LED beads 152. The LED beads 152 are positioned on a surface of the lamp board 150 opposite the inlet section 102. A button 158 is mounted on the housing 130, and the button 158 and the battery 154 are electrically connected to the lamp board 150, enabling user control of the on/off state of the light source 148. The use of multiple LED beads 152 increases the luminous intensity of the light source 148 and improves the overall phototherapy effect. Optionally, the plurality of LED beads 152 may be arranged in a ring-shaped array to enhance uniformity of illumination. The LED beads 152 may include red LED beads, blue LED beads, infrared LED beads, or multi-core LEDs to support diverse therapeutic functions.

Optionally, the housing 130 is fitted with a keycap 146. The keycap 146 is configured to press the button 158 to switch the light source 148 on or off. The keycap 146 may be made of rubber or silicone and may be fixed to the housing 130 by injection molding to provide sealing around the corresponding region of the button 158. In alternative embodiments, the button 158 may be implemented as a pressure switch, a touch switch, or another switching mechanism adapted to facilitate waterproofing and sealing of the device.

In one embodiment, the light source 148 (light-emitting elements) further includes a lens 156. When multiple LED beads 152 are provided, a corresponding plurality of lenses 156 may be included, with each lens 156 aligned to a respective LED bead 152. The lens 156 is configured to regulate the illumination range of each LED bead 152, thereby enabling a more uniform distribution of emitted light.

In an embodiment, the plurality of LED beads 152 may be arranged in a ring-shaped array comprising 6, 8, 12, 16, or more individual LED beads distributed uniformly around the circumference of the lamp board 150. The LED beads 152 may be spaced at equal angular intervals, such as every 30 degrees, 45 degrees, or 60 degrees, to provide uniform illumination coverage. The LED beads 152 may all emit the same wavelength, or may include a combination of different wavelengths, such as alternating red and infrared LEDs, or a mixture of red, blue, and infrared LEDs arranged in a predetermined pattern to provide multi-wavelength therapeutic treatment.

In an embodiment, the lens 156 may be formed from a transparent or translucent material such as optical-grade glass, acrylic, polycarbonate, or silicone. The lens 156 may be configured as a convex lens, concave lens, Fresnel lens, or diffuser element, depending on the desired light distribution pattern. The lens 156 may have a focal length ranging from 5 mm to 50 mm, and may be configured to produce a beam angle ranging from 15 degrees to 120 degrees. The lens 156 may be designed to converge the light emitted by the LED bead 152 into a focused beam for deep tissue penetration, or to diffuse the light into a wide-angle pattern for broad-area coverage, depending on the therapeutic application.

In an embodiment, the phototherapy unit 126 is configured to operate using therapeutic light within a broad wavelength range of approximately 630 nm to 850 nm, enabling the device to support diverse photo-biomodulation functions suited for skin health, circulation enhancement, and inflammatory reduction during shower use. In an embodiment, the light source may be optimized to emit within a more targeted range of 650 nm to 850 nm, which has been shown to provide improved penetration depth and enhanced biological response for skin revitalization and tissue support. The phototherapy unit 126 can include a plurality of built-in light-emitting diodes (LEDs) 152, comprising 12 LEDs configured to emit therapeutic light at two different wavelengths, including approximately 650 nm and approximately 850 nm independently or simultaneously. These wavelength selections may be achieved through corresponding LED beads 152, lens 156 configurations, or driving circuitry parameters, and may be presented individually or in combination, depending on the selected user mode. The integration of these wavelength ranges allows the multifunctional shower head to deliver consistent and effective therapeutic irradiation concurrently with water flow.

In an embodiment, the phototherapy unit 126 may further include safety features configured to protect the user and the device during operation. The control circuitry may include an automatic shut-off timer configured to deactivate the light source after a predetermined treatment duration, which may range from 5 minutes to 30 minutes. The phototherapy unit 126 may further include a temperature sensor disposed adjacent to the lamp board 150 or LED beads 152, configured to monitor the operating temperature and reduce power or shut off the light source if the temperature exceeds a predetermined threshold. The housing 130 may further include heat dissipation structures such as fins, vents, or thermally conductive materials to facilitate cooling of the LED beads 152 during extended operation.

In an embodiment, the phototherapy unit 126 may include electrical isolation features configured to prevent electrical current from reaching water-carrying components of the shower body 100. The housing 130 may be formed from electrically insulating materials, and all electrical connections may be sealed within the waterproof housing 130. The phototherapy unit 126 may be configured to operate at low voltage, such as 5V, 12V, or 24V DC, to enhance user safety in wet environments. The device may further include ground fault protection circuitry or equivalent safety mechanisms configured to detect electrical leakage and shut off power in the event of a fault condition. The phototherapy unit 126 may be designed to comply with applicable electrical safety standards for wet environments, such as IPX7 or IPX8 waterproof ratings.

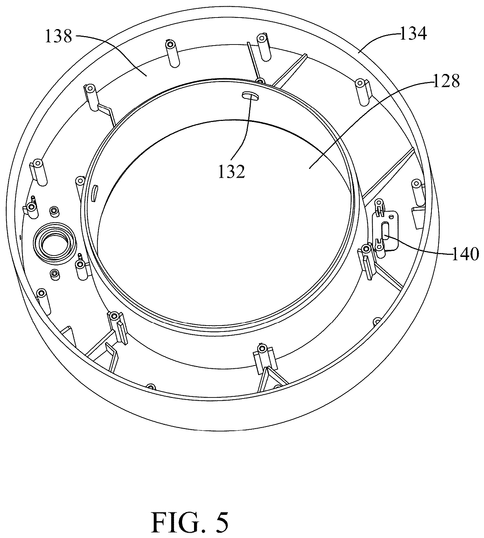

Referring to **FIGS.** 2 to 5, in an embodiment, the housing 130 comprises a mounting shell 134 and a cover plate 136. The mounting shell 134 is annular in shape, and the cover plate 136 is likewise annular. A mounting hole 128 is formed at the center of the mounting shell 134. An annular groove 138 is defined on the side of the mounting shell 134 facing toward the outlet hole 108. The light source 148 and the battery 154 are disposed within the annular groove 138. The cover plate 136 is a light-transmitting component that covers and seals the annular groove 138. The cover plate 136 may be formed with a plurality of cutouts corresponding to the respective positions of the LED beads 152 and the lenses 156, thereby allowing light to be emitted and directed toward a target area.

The outlet hole 108 may be arranged such that it avoids the mounting hole 128 and the cover plate 136, which simplifies the manufacturing of the housing 130 and facilitates effective sealing of the light source 148 and battery 154 inside the annular groove 138. This prevents water vapour from entering the housing 130 during water discharge from the shower body 100. Optionally, the cover plate 136 may be formed from glass or transparent silicone to improve light transmission, maintain luminous output, and enhance the phototherapy effect. The cover plate 136 and the mounting shell 134 may be sealed by adhesive bonding, welding, or other sealing methods to ensure airtightness.

In an embodiment, the mounting shell 134 may be formed from a water-resistant polymeric material such as acrylonitrile butadiene styrene (ABS), polycarbonate (PC), polyethylene terephthalate (PET), or a combination thereof. The mounting shell 134 may further include additives or coatings to enhance UV resistance, corrosion resistance, or antimicrobial properties. The shower body 100 may be formed from similar materials or may alternatively be formed from stainless steel, brass, chrome-plated metal, or other corrosion-resistant materials suitable for prolonged water exposure.

Additionally, the housing 130 further includes a ring frame 144, which is coupled to the lamp board 150 and/or the mounting shell 134. The lens 156 is clamped between the lamp board 150 and the ring frame 144, with the ring frame 144 positioned between the cover plate 136 and the annular groove 138. This structure facilitates the stable positioning of the lens 156 and the light source 148. The ring frame 144 may be connected to the lamp board 150 or the mounting shell 134 via screws, clips, or similar fastening components to enable convenient disassembly and maintenance.

In an embodiment, a bracket 160 is further provided within the housing 130, and a lens 156 is mounted inside the bracket 160. The bracket 160 may be formed as a cylindrical or tubular structure configured to receive and retain the lens 156 in axial alignment with a corresponding LED bead 152. Opposite ends of the bracket 160 respectively abut the lamp board 150 and the ring frame 144, thereby maintaining a fixed spacing between the LED bead 152 and the lens 156. The bracket 160 may be formed from a rigid polymeric material, metal, or composite material, and may include internal retention features such as annular grooves, ribs, or snap-fit elements to secure the lens 156 against axial or lateral displacement. Multiple brackets 160 may be provided in a circular array, with each bracket 160 corresponding to one LED bead 152 and one lens 156. This arrangement stabilizes the positional alignment of the lens 156, ensuring that each LED bead 152 remains properly aligned with its corresponding lens 156 throughout operation and during exposure to vibration or thermal expansion.

Referring to **FIGS. 2** to **4****,** in an embodiment, the connecting position 110 is configured as a slot, and the locking position 132 is configured as a protrusion. The protrusion engages the slot to lock the locking position 132 to the connecting position 110, thereby facilitating convenient connection and disassembly between the shower body 100 and the phototherapy unit 126. In alternative embodiments, the connecting position 110 may be formed as a protrusion and the locking position 132 as a slot. In such cases, the slot-protrusion engagement similarly enables the outlet section 106 of the shower body 100 to be securely locked to the phototherapy unit 126.

Referring to **FIGS. 1** to **4****,** in an embodiment, the mounting hole 128 is formed as a circular hole, and the outlet section 106 is cylindrical. The connecting position 110 includes a first groove 112, a second groove 114, and a locking groove 116. The first groove 112 is arranged along the axial direction of the outlet section 106, while the second groove 114 is arranged circumferentially around the outlet section 106. The locking groove 116 is positioned to receive and retain the protrusion of the locking position 132. The first groove 112 extends axially from an end of the outlet section 106 near the outlet hole 108 to an intermediate portion of the outlet section 106. Opposite ends of the second groove 114 are respectively connected to the first groove 112 and the locking groove 116.

To connect the shower body 100 to the phototherapy unit 126, the outlet section 106 is inserted into the mounting hole 128. The locking protrusion moves from the open end of the first groove 112 toward the opposite end of the first groove 112 near the second groove 114. Upon reaching this location, the phototherapy unit 126 is rotated, causing the locking protrusion to slide along the second groove 114 toward the end of the second groove 114 adjacent the locking groove 116. When the locking protrusion enters and seats in the locking groove 116, the locking groove 116 securely engages the protrusion, thereby preventing disengagement between the shower body 100 and the phototherapy unit 126. This structure ensures stable and reliable attachment.

Optionally, the locking groove 116 may be elongated, extending from the end of the second groove 114 away from the first groove 112 toward the end of the outlet section 106 adjacent the outlet hole 108. The depth of the end of the locking groove 116, farthest from the second groove 114, may be greater than the depth of the second groove 114. This configuration further prevents the locking protrusion from inadvertently sliding out of the locking groove 116 and re-entering the second groove 114, thereby preventing loosening of the phototherapy unit 126 during use.

In an embodiment, the phototherapy unit 126 further comprises a control circuit board disposed within the housing and electrically connected to the light source, the control circuit board being configured to regulate power delivery, light intensity, wavelength selection, and operational timing of the light source. The control circuit board may include a microcontroller, LED driver circuits, power management circuitry, and input processing components configured to receive signals from the button or wireless receiver module and adjust the operational parameters accordingly.

In an embodiment, the sealing member comprises an O-ring, gasket, or waterproof membrane disposed between the shower body and the phototherapy unit, the sealing member being configured to compress when the phototherapy unit is locked to the outlet section, thereby forming a watertight seal that prevents water ingress into the electrical components of the phototherapy unit during high-pressure shower operation.

Referring to **FIGS. 2** to **4****,** in an embodiment, the battery 154 is rechargeable, and the housing 130 is provided with a through hole 140. A charging port 162 electrically connected to the light source 148 is installed within the through hole 140, and a sealing cover 142 is mounted on the housing 130 to seal the through hole 140. Optionally, the charging port 162 may be a TYPE-C connector, which facilitates convenient recharging of the battery 154 and supports environmentally friendly reuse. Specifically, the lamp board 150 is electrically connected to the charging port 162.

In an embodiment, the phototherapy unit 126 is detachably coupled to the shower head. When charging is required, the phototherapy unit can be removed from the shower and charged independently of the shower head.

In one embodiment of this application, the housing 130 is further provided with a charging electrode electrically connected to the light source 148. The charging electrode protrudes outward from the housing 130 and may be integrally injection-molded and fixed to the housing 130. The charging electrode may be implemented as a magnetic electrode, conductive post, conductive sheet, or other conductive structure. This configuration enhances the sealing capability of the charging interface and improves circuit safety. The lamp board 150 is electrically connected to the charging electrode.

In one embodiment of this application, a wireless charging coil electrically connected to the light source 148 is installed within the housing 130. The wireless charging coil enables sealed, contactless charging of the phototherapy unit 126, thereby allowing the entire circuit to remain enclosed within the housing 130. This improves waterproof performance and overall circuit safety. Specifically, the lamp board 150 is electrically connected to the wireless charging coil.

In one embodiment, the charging electrode may comprise a pair of conductive contacts disposed on the outer surface of the housing 130, positioned to align with corresponding contacts on a charging base or docking station. The charging electrodes may be formed from corrosion-resistant conductive materials such as gold-plated copper, stainless steel, or conductive polymers. The charging electrodes may be recessed within the housing 130 to prevent accidental contact during normal use, and may be surrounded by insulating material to prevent water ingress. When the phototherapy unit 126 is placed on the charging base, the electrodes make physical contact to establish an electrical connection for charging the battery 154. The charging electrodes may incorporate magnetic alignment features to ensure proper positioning and contact pressure during charging.

The rechargeable battery 154 configured to retain sufficient charge to support at least 10 treatment cycles, each treatment cycle having a duration of approximately 20 minutes.

An indicator can be integrated on a side portion of the phototherapy unit 126 and is configured to illuminate when the phototherapy unit 126 is powered on. When the battery power level is sufficient, the indicator emits a blue light, and when the battery power level is insufficient, the indicator emits a white light flashing at a frequency of approximately 1 Hz to provide a charging warning to the user. The indicator emits a green light, once the phototherapy unit 126 is fully charged.

In one embodiment of this application, the inlet section 102 and the outlet section 106 are detachably connected, and a filter 118 is disposed within the inlet section 102. The filter 118 filters and buffers the incoming water, ensuring cleaner water flow and promoting a more stable output during use. Optionally, the inlet section 102 and the outlet section 106 may be thread-connected, which enhances sealing performance at the joint and prevents leakage.

The filter can include a plurality of functional filtration layers which can comprise can be a 304 stainless steel filter screen forming a blocking layer configured to intercept silt, rust, and large particulate impurities; a high-precision polypropylene (PP) cotton filter layer configured to remove suspended solids and fine particulate matter; an alkaline sphere adjustment layer configured to regulate water pH, release beneficial trace minerals, and generate negative ions; a United States KDF (USKDF) bactericidal sterilization layer configured to remove residual chlorine, heavy metals, and inhibit microbial growth; a ceramic ball improvement layer configured to reduce residual chlorine, retain beneficial minerals, and improve water taste; an activated carbon adsorption layer configured to adsorb residual chlorine, odors, discoloration, and organic contaminants; a calcium sulfite residual chlorine removal layer configured to rapidly neutralize chlorine, chlorinated by-products, and rust; and a mineralized medical stone (maifanite) layer configured to release beneficial trace elements into the water. Collectively, the filtration layers cooperate to purify, mineralize, sterilize, and condition water delivered through the shower.

In one embodiment of this application, the outlet section 106 is provided with a cover 124, a water distribution cover 120, and a center cover 122. The cover 124 is mounted over the water outlet end of the outlet section 106, defining a receiving cavity between the cover 124 and the outlet section 106. A through hole is formed at the center of the cover 124.

The water distribution cover 120 is positioned outside the cover 124 and is configured to regulate the water flow. A process hole is formed at the center of the water distribution cover 120, and the center cover 122 is mounted over the process hole to seal the through hole. The receiving cavity may be filled with purified water particles, which serve to slow and stabilize the water flow, thereby producing a more uniform water output. The water distribution cover 120 allows convenient adjustment of the water flow and facilitates uniform lateral distribution. The outlet hole 108 is formed in the water distribution cover 120, which is rotatably mounted within the outlet section 106 to enable adjustable water dispersion.

The part of the shower head where the water outlet holes are located can be formed from a silicone material and includes a silicone pad defining a plurality of water outlet holes 108. The silicone construction provides flexibility and resilience, enabling easy removal of limescale and mineral deposits by manual wiping, while also improving durability, anti-clogging performance, and uniform water dispersion during operation.

In one embodiment, the water flow path through the multifunctional shower head may be described as follows: water enters the inlet section 102 through the inlet hole 104 and passes through the filter 118, which removes impurities and sediments. The filtered water then flows into the outlet section 106, where it enters the receiving cavity defined between the cover 124 and the outlet section 106. Within the receiving cavity, the water may contact purified water particles or flow-regulating elements that slow and stabilize the flow. The water then passes through the water distribution cover 120, which directs the water toward the multiple outlet holes 108 arranged in an annular pattern around the phototherapy unit 126. The outlet holes 108 are positioned to avoid the mounting hole 128 and the cover plate 136 of the phototherapy unit 126, allowing water to be discharged in a spray pattern that surrounds the therapeutic light emitted by the phototherapy unit 126. This arrangement enables simultaneous delivery of water and therapeutic light to the user's skin without interference between the water flow and the light emission.

In one embodiment, the multifunctional shower head may be assembled by first connecting the inlet section 102 to the outlet section 106, with the filter 118 installed within the inlet section 102. The water distribution cover 120, the center cover 122, and cover 124 may then be sequentially mounted to the outlet section 106. The phototherapy unit 126 may be assembled separately by installing the lamp board 150, LED beads 152, lenses 156, battery 154, and associated circuitry within the annular groove 138 of the mounting shell 134, followed by sealing the cover plate 136 to the mounting shell 134 through adhesive bonding, ultrasonic welding, or thermal fusion. The assembled phototherapy unit 126 may then be attached to the shower body 100 by inserting the outlet section 106 through the mounting hole 128 and rotating the phototherapy unit 126 to engage the locking position 132 with the connecting position 110. Quality control procedures may include waterproof testing, electrical safety testing, light output measurement, and mechanical durability testing to ensure compliance with applicable standards.

In one embodiment of this application, multiple connection positions 110 are arranged in a circular array around the outlet section 106. Correspondingly, multiple locking positions 132 are provided on the phototherapy unit 126, each arranged in a one-to-one relationship with a corresponding connection position 110. This distributed locking structure enhances the stability of the connection between the shower body 100 and the phototherapy unit 126, ensuring reliable engagement during operation.

In one embodiment of the present invention, the LED ring of the phototherapy unit 126 is provided with a comprehensive waterproofing structure configured to protect the internal light-emitting components during continuous exposure to shower water.

Referring to **FIGS. 2** to **5****,** in an embodiment, the mounting shell 134 and the light-transmitting cover plate 136 are sealed together to enclose the annular groove 138 in a waterproof manner. A peripheral waterproof gasket is disposed between the mounting shell 134 and the cover plate 136, and the gasket is compressed uniformly along the circumference of the annular groove 138 to prevent water, steam, or moisture from entering the interior of the housing 130. The sealing interface between the mounting shell 134 and the cover plate 136 may further be reinforced through adhesive bonding, ultrasonic welding, or thermal fusion, thereby forming a rigid waterproof barrier around the lamp board 150 and the plurality of lenses 156. Additionally, the lamp board 150 may be coated with a conformal waterproof insulating layer to prevent corrosion of the LED beads 152 and associated circuitry, and the ring frame 144 positioned above the lamp board 150 further stabilizes the lenses 156 while maintaining a controlled compression on the sealing surfaces. Through this multi-layered sealing arrangement, the LED ring is protected against water ingress during high-pressure shower operation, ensuring long-term safety, durability, and illumination stability of the phototherapy unit 126.

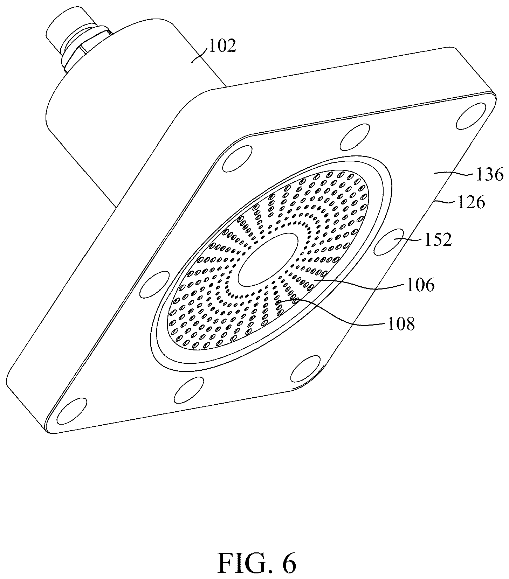

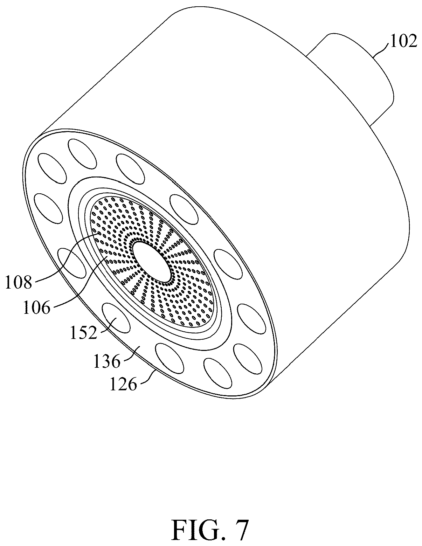

Referring to **FIGS. 6** and **7****,** in an embodiment, the shower head may be formed in different shapes while retaining the same functional structure and therapeutic capabilities. The external housing profile of the shower body 100 and the phototherapy unit 126 may be adapted to various geometric configurations, including square, triangular, rectangular, oval, polygonal, or custom-contoured forms to suit aesthetic preferences or ergonomic requirements. When the housing is formed in a square configuration, the inlet section 102, outlet section 106, and connecting position may be arranged along the central axis of the square profile, with the phototherapy unit 126 configured as a square annular frame surrounding the outlet section 106. The outlet holes 108 may be distributed in a pattern adapted to the square geometry, such as arranged along the four sides or in a grid pattern. Similarly, when the housing is formed in a rectangular configuration, the components may be elongated along one axis while maintaining the same attachment interface and functional arrangement. The locking mechanism, comprising the connecting position and locking position, remains structurally and functionally identical regardless of the external housing shape, ensuring consistent detachability and secure engagement. The shape variation does not affect the internal water flow path, the sealing performance of the phototherapy unit 126, the light emission characteristics of the LED beads 152, the cover plate 136, or the therapeutic effectiveness of the device.

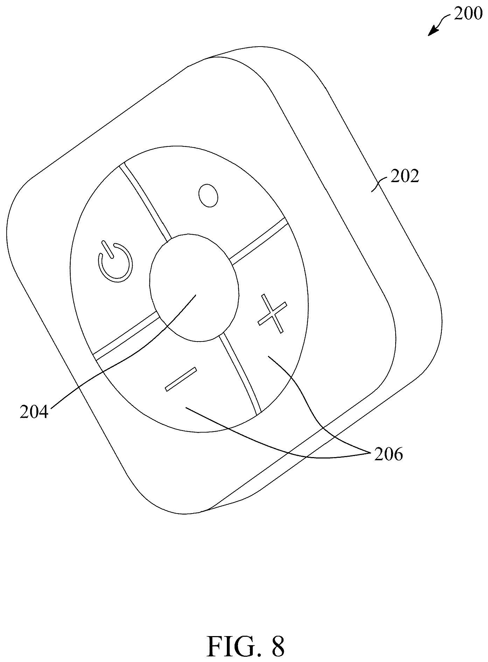

Referring to **FIG. 8****,** in an embodiment, the shower head further includes a waterproof remote-control unit 200 configured to wirelessly operate the various therapeutic functions of the phototherapy unit. The remote-control unit 200 comprises a compact remote housing 202 having a rounded-square profile and a front-facing display window 204 positioned centrally on the housing. The display window 204 is configured to present operational information, including the selected light mode, intensity level, and remaining therapy duration. Surrounding the display window 204 is a control interface formed by a plurality of integrated buttons 206 disposed on the surface of the remote housing 202. These include a power button for switching the phototherapy unit on or off, a mode-selection button for toggling between different therapeutic light wavelengths or pre-set programs, and a pair of intensity-adjustment buttons, namely an increase button and a decrease button, for regulating the luminous output of the LED array.

During operation, the remote-control unit 200 communicates wirelessly with the phototherapy device to adjust functional parameters in real time. Activation of the mode-selection button enables the user to cycle through available phototherapy modes such as red light, blue light, infrared light, or combined wavelength programs. The intensity-adjustment buttons allow precise control over light output, enabling the user to tailor therapy strength to personal preference or skin sensitivity. The remote-control unit 200 further incorporates a duration-setting interface, whereby the user may set or modify treatment time intervals displayed on the display window 204. The low-profile sealed buttons and compact construction of the remote housing 202 ensure moisture resistance and stable operation even in humid or wet environments, allowing the user to conveniently regulate all therapeutic parameters without directly manipulating the main shower-head assembly.

In an embodiment, the remote-control unit 200 communicates with the phototherapy unit 126 through a wireless communication protocol, which may include Bluetooth, radio frequency (RF), infrared (IR), or other suitable wireless transmission methods. The phototherapy unit 126 may include a corresponding wireless receiver module disposed within the housing 130 and electrically connected to the lamp board 150 and control circuitry. The wireless receiver module may be configured to receive command signals from the remote-control unit 200 and adjust the operational parameters of the LED beads 152 accordingly. The remote-control unit 200 may be powered by a replaceable or rechargeable battery enclosed within the remote housing 202.

When the power button 158 on the phototherapy unit 126 is pressed for a short duration, the phototherapy unit 126 is powered on and placed in an active standby mode without initiating phototherapy operation. In this active mode, the device remains powered but inactive, and initiation and control of the working state are performed via a remote control by short-press actuation of buttons 206 provided on the remote-control unit 200.

The present invention provides a multifunctional shower head capable of integrating therapeutic light delivery, adjustable water-spray performance, and modular user-operated features within a compact and adaptable structure. The detachable nature of the waterproof phototherapy unit, the remote-control interface, and the ability to construct the shower head in multiple shapes enable broad customization without compromising functionality. The invention therefore provides a highly versatile system that enhances user comfort, promotes therapeutic benefits, and simplifies maintenance and operation. Although specific embodiments have been described, it will be understood that various modifications may be implemented without departing from the spirit and scope of the invention.

From an industrial perspective, the invention is well-suited for large-scale manufacture and integration into consumer bath fixtures, wellness systems, professional therapy facilities, smart-home installations, and hotel or spa environments. The modular design, standardized interfaces, and robust waterproof construction make the device compatible with existing plumbing infrastructure and adaptable to diverse commercial and residential markets. The manufacturing process may utilize automated assembly lines for high-volume production, with quality control checkpoints at critical stages including waterproof sealing, electrical testing, and light output calibration. The device may be manufactured at competitive costs due to the use of standard electronic components, injection-molded plastic housings, and established LED technology. Market applications include residential bathrooms for daily wellness routines, commercial spa and salon facilities for professional treatments, medical clinics for therapeutic phototherapy sessions, and hospitality environments such as hotels and resorts seeking to provide enhanced guest amenities. The device may be designed to comply with relevant regulatory standards, including electrical safety certifications (UL, CE, FCC), waterproof ratings (IPX7/IPX8), and medical device regulations where applicable (FDA Class II for therapeutic devices). The modular and detachable design facilitates maintenance, repair, and component replacement, extending product lifespan and reducing long-term ownership costs.

Various modifications to these embodiments are apparent to those skilled in the art from the description and the accompanying drawings. The principles associated with the various embodiments described herein may be applied to other embodiments. Therefore, the description is not intended to be limited to the embodiments shown along with the accompanying drawings but is to provide the broadest scope consistent with the principles and the novel and inventive features disclosed or suggested herein. Accordingly, the invention is anticipated to hold on to all other such alternatives, modifications, and variations that fall within the scope of the present invention and appended claims.

## Claims

1. A phototherapy unit configured for retrofit installation on a shower head, comprising:
a housing defining a central opening sized to receive an outlet portion of the shower head;
a plurality of light-emitting elements disposed within the housing and oriented to emit therapeutic light toward a user during shower operation;
a locking mechanism disposed on the housing and configured to detachably secure the phototherapy unit onto an exterior surface of the shower head; and
wherein the phototherapy unit mounts to the shower head without structural modification of the shower head.

2. The phototherapy unit of claim 1, wherein the housing is selected from a circular, oval, polygonal, square, triangular or rectangular shape to accommodate shower heads having different geometries.

3. The phototherapy unit of claim 1, wherein the housing includes a region supporting a lamp board, lenses, and a transparent cover plate.

4. The phototherapy unit of claim 1, wherein the locking mechanism comprises at least one groove, slot, ridge, or tab configured to engage a complementary locking feature on the shower head.

5. The phototherapy unit of claim 1, wherein the plurality of light-emitting elements emit wavelengths in the range of 630 to 850 nm.

6. The phototherapy unit of claim 1, wherein the locking mechanism comprises engagement features configured for insertion and rotation to secure the phototherapy unit to the shower head.

7. The phototherapy unit of claim 1, wherein the phototherapy unit is detachable for charging or maintenance independently of the shower head.

8. A shower head assembly comprising:
a shower head having an inlet portion, an outlet portion, and a plurality of water outlet holes located at a front face of the outlet portion;
a phototherapy unit detachably coupled to the outlet portion, the phototherapy unit including a housing surrounding the shower head;
wherein the phototherapy unit comprises a plurality of light-emitting elements; and
wherein the shower head and the phototherapy unit are configured to deliver the phototherapy during a shower.

9. The shower head assembly of claim 8, wherein the phototherapy unit includes a mounting shell defining a groove for seating the light-emitting elements.

10. The shower head assembly of claim 8, wherein the outlet portion includes a connection position formed with a first guide groove, a second guide groove, and a locking groove.

11. The shower head assembly of claim 10, wherein the phototherapy unit includes a locking protrusion configured to enter the first guide groove, travel into the second guide groove, and seat in the locking groove during rotational engagement.

12. The shower head assembly of claim 11, wherein multiple locking protrusions and multiple connection positions are circumferentially arranged to increase mounting stability.

13. The shower head assembly of claim 8, wherein the light-emitting elements are mounted on a metal-core circuit board for improved thermal management.

14. The shower head assembly of claim 8, wherein the phototherapy unit includes one or more lenses aligned one-to-one with the light-emitting elements to focus emitted light.

15. A phototherapy unit for a shower head, comprising:
a housing configured to be mounted on or integrated with an outlet portion of the shower head;
a lamp board having a plurality of light-emitting elements disposed in the housing;
a power supply electrically connected to the light-emitting elements;
a waterproofing structure enclosing the power supply and an electrical circuitry within the housing;
wherein the waterproofing structure prevents water ingress into the electrical circuitry during shower operation;
wherein the outlet portion includes a connection position formed with a first guide groove, a second guide groove, and a locking groove; and
wherein the phototherapy unit includes a locking protrusion configured to enter the first guide groove, travel into the second guide groove, and seat in the locking groove during rotational engagement.

16. The phototherapy unit of claim 15, wherein the housing enables axial insertion of the outlet portion followed by rotational locking.

17. The phototherapy unit of claim 15, wherein the waterproofing structure comprises at least one sealing ring, gasket, adhesive bonding layer, ultrasonic-welded joint, or conformal coating applied to the lamp board.

18. The phototherapy unit of claim 15, wherein the housing includes a transparent cover plate formed of glass, polycarbonate, or silicone.

19. The phototherapy unit of claim 15, wherein the power supply comprises a rechargeable battery enclosed within the waterproofing structure.

20. The phototherapy unit of claim 15, further comprising a wireless charging coil disposed within the housing.
